(19) Europäisches Patentamt
European Patent Office
Office européen des brevets

(11) **EP 4 394 354 A1**

(12) **EUROPEAN PATENT APPLICATION**
published in accordance with Art. 153(4) EPC

(43) Date of publication:
**03.07.2024 Bulletin 2024/27**

(21) Application number: **22860849.3**

(22) Date of filing: **18.03.2022**

(51) International Patent Classification (IPC):
***G01N 21/27*** *(2006.01)*

(52) Cooperative Patent Classification (CPC):
**G01N 33/1846; G01N 21/27**

(86) International application number:
**PCT/JP2022/012747**

(87) International publication number:
**WO 2023/026551 (02.03.2023 Gazette 2023/09)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.08.2021 JP 2021138153**

(71) Applicant: **Sony Group Corporation
Tokyo 108-0075 (JP)**

(72) Inventors:
• **TAKATSUKA, Susumu
Tokyo 108-0075 (JP)**
• **TETSUKAWA, Hiroki
Tokyo 108-0075 (JP)**

(74) Representative: **MFG Patentanwälte
Meyer-Wildhagen Meggle-Freund
Gerhard PartG mbB
Amalienstraße 62
80799 München (DE)**

(54) **MEASUREMENT DEVICE, MEASUREMENT METHOD, AND PROGRAM**

(57) A measurement device includes an image capture control unit that causes an image capture unit to capture an image of a predetermined image capture range in water, and a carbon amount estimation unit that estimates an amount of carbon based on the image captured by the image capture unit.

Fig. 1

EP 4 394 354 A1

## Description

[Technical Field]

The present technique relates to a measurement device, a measurement method, and a program, and relates particularly to a technique for measuring an amount of carbon in water.

[Background Art]

A measurement device has been proposed which measures the abundance of phytoplankton by irradiating phytoplankton with excitation light at a predetermined wavelength to excite the phytoplankton and then measuring the intensity of fluorescence emitted from the excited phytoplankton (see PTL 1, for example).

[Citation List]

[Patent Literature]

[PTL 1]
JP 2019-165687A

[Summary]

[Technical Problem]

Incidentally, to reduce greenhouse gases that contribute to global warming, it is necessary to observe changes in carbon, which is the source of the greenhouse gas carbon dioxide, over the long term. Some carbon dioxide is absorbed by oceans (into water). Some of the carbon dioxide absorbed by oceans is also captured as carbon by phytoplankton through photosynthesis. Furthermore, the carcasses and feces of phytoplankton, zooplankton that preyed on phytoplankton, and the like accumulate in deep waters as marine snow, which acts as a carbon sink effective for thousands of years.

However, the above-described measurement device can only measure phytoplankton excited by excitation light. In addition, although the above-described measurement device can measure the abundance of phytoplankton, the measurement device cannot measure an amount of carbon.

Accordingly, an object of the present technique is to estimate an amount of carbon.

[Solution to Problem]

A measurement device according to the present technique includes an image capture control unit that causes an image capture unit to capture an image of a predetermined image capture range in water, and a carbon amount estimation unit that estimates an amount of carbon based on the image captured by the image capture unit.

This makes it possible for the measurement device to estimate an amount of carbon based on an image captured by the image capture unit.

[Brief Description of Drawings]

[Fig. 1]

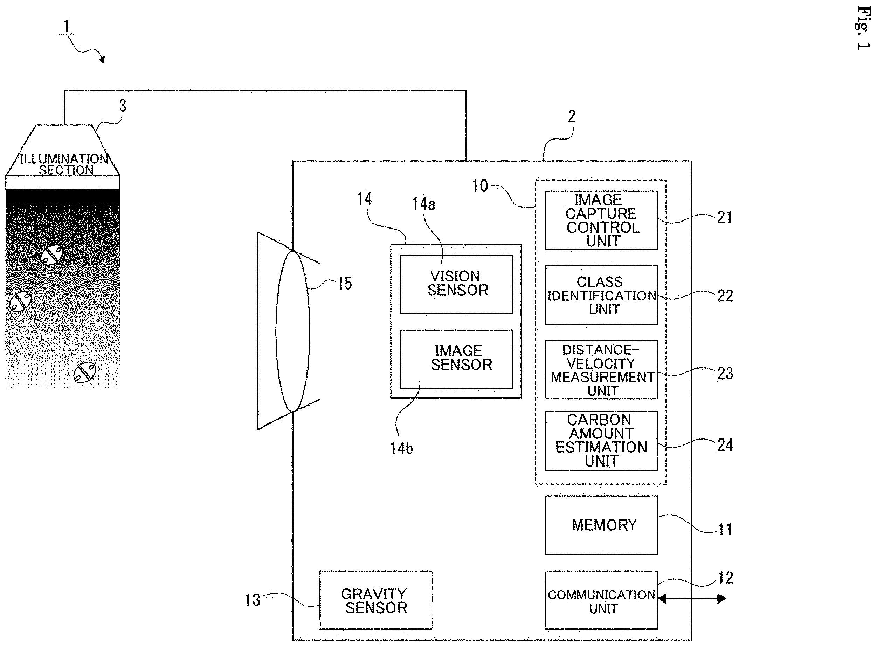

Fig. 1 is a diagram illustrating the configuration of a measurement device serving as a first embodiment.
[Fig. 2]

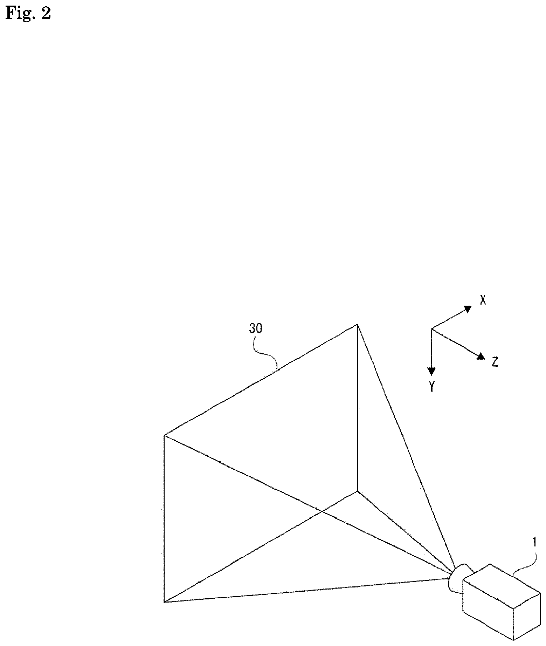

Fig. 2 is a diagram illustrating an image capture range and a measurement direction.
[Fig. 3]

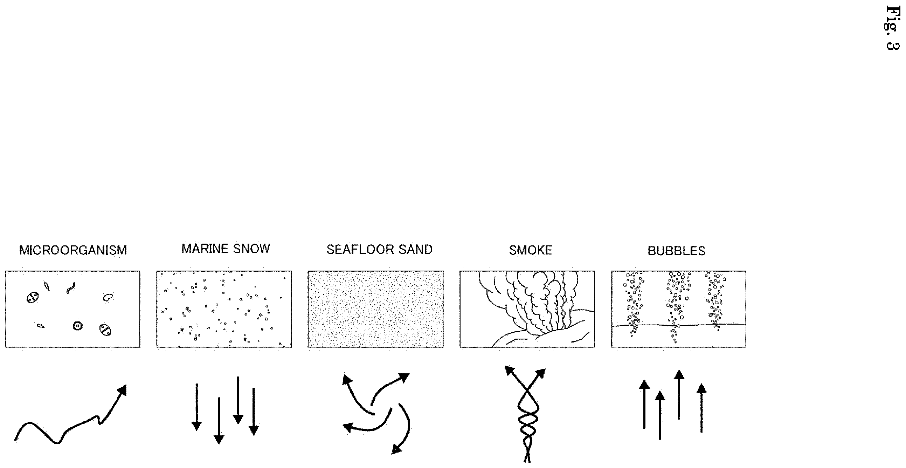

Fig. 3 is a diagram illustrating a target object and movement of a target object.
[Fig. 4]

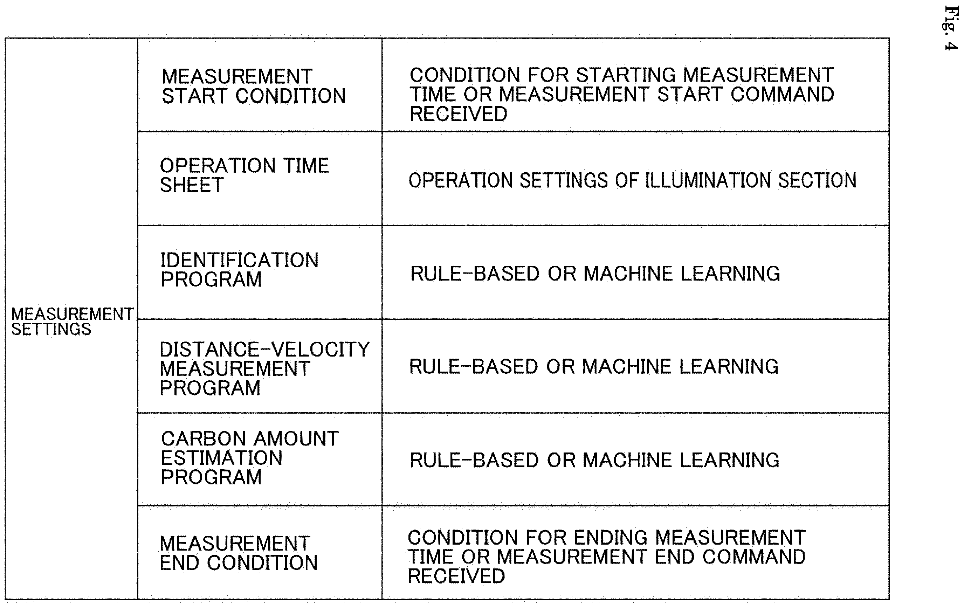

Fig. 4 is a diagram illustrating an example of measurement settings.
[Fig. 5]

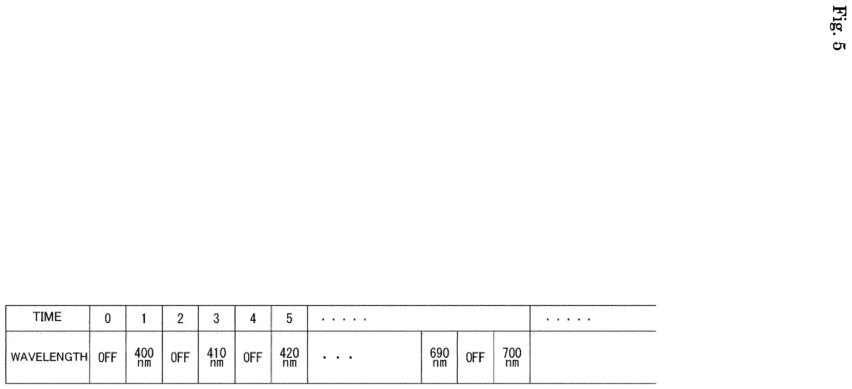

Fig. 5 is a diagram illustrating an example of an operation time sheet.
[Fig. 6]

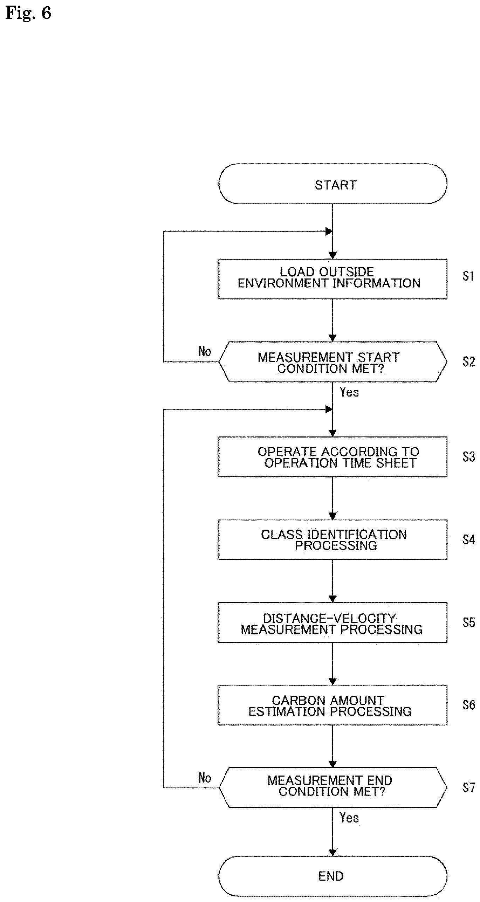

Fig. 6 is a flowchart illustrating a sequence of measurement processing.
[Fig. 7]

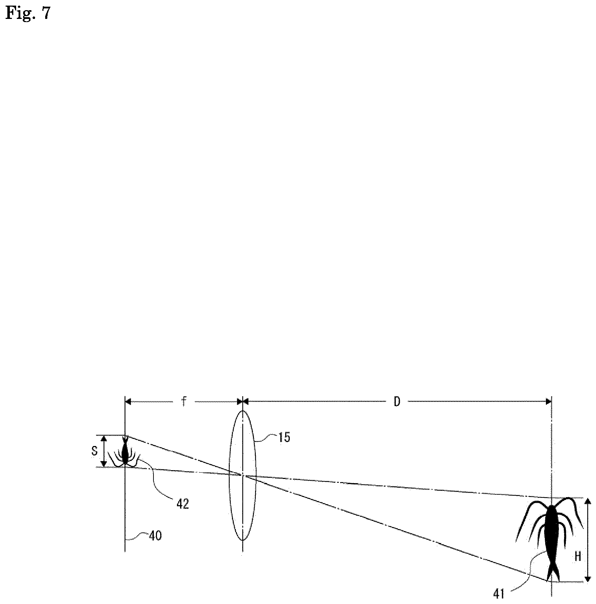

Fig. 7 is a diagram illustrating rule-based distance-velocity measurement processing.
[Fig. 8]

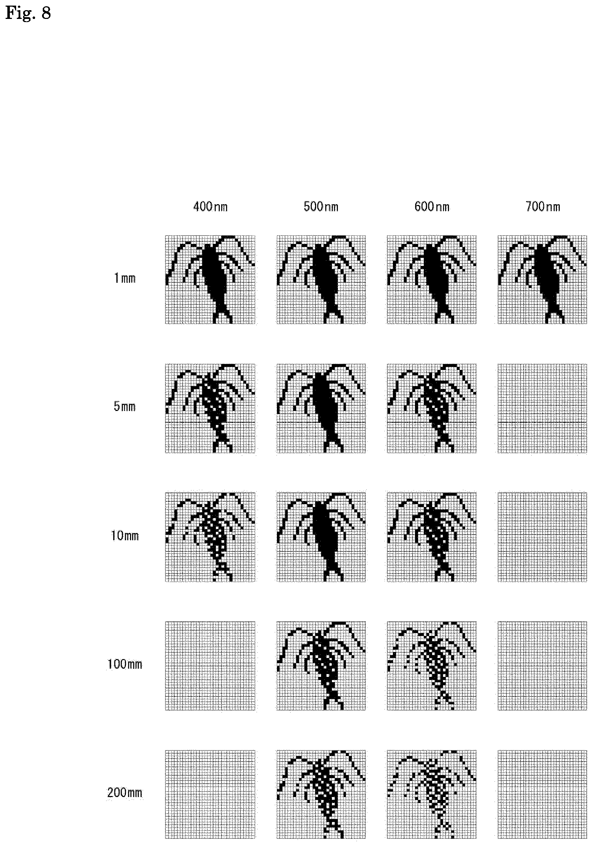

Fig. 8 is a diagram illustrating images serving as supervisory data.
[Fig. 9]

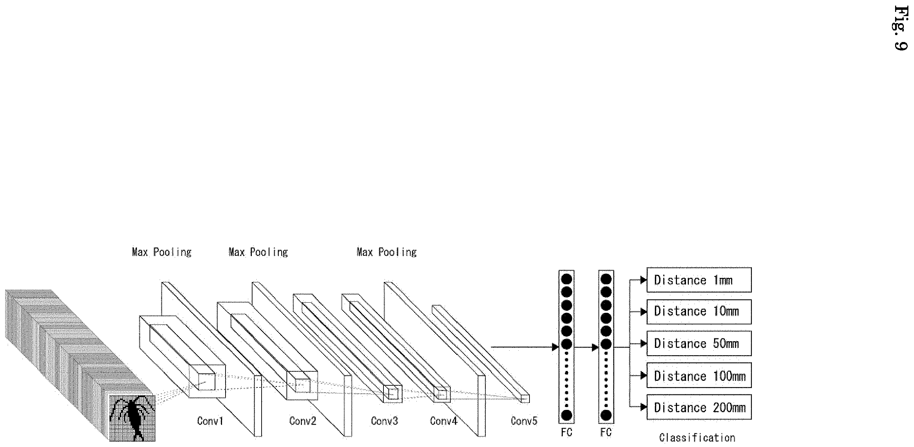

Fig. 9 is a diagram illustrating a deep learning model.
[Fig. 10]

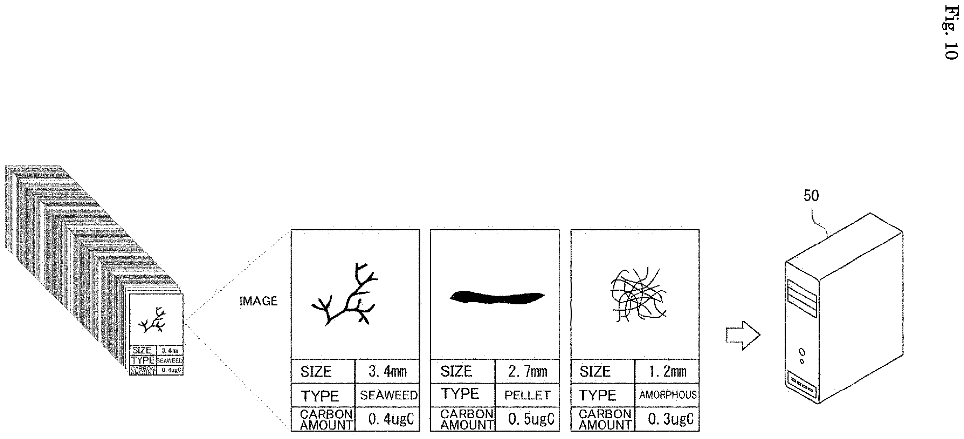

Fig. 10 is a diagram illustrating machine learning for carbon amount estimation processing.
[Fig. 11]

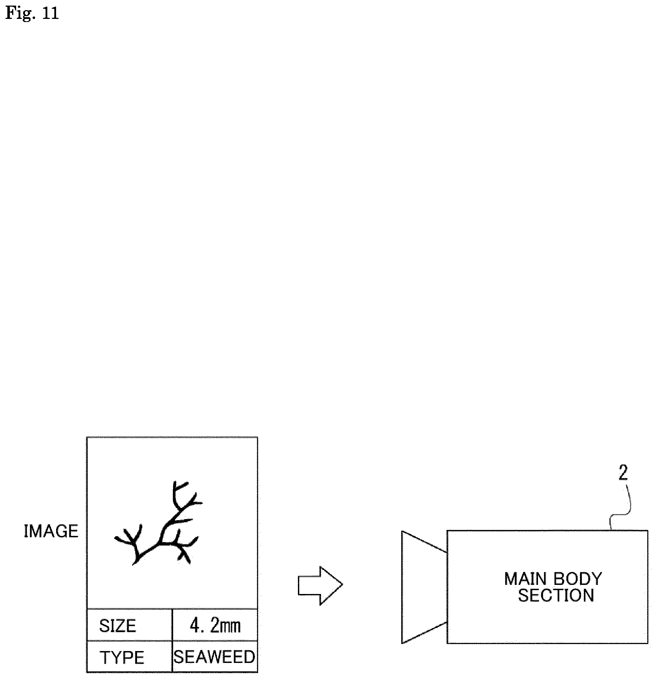

Fig. 11 is a diagram illustrating spatial carbon amount estimation processing.
[Fig. 12]

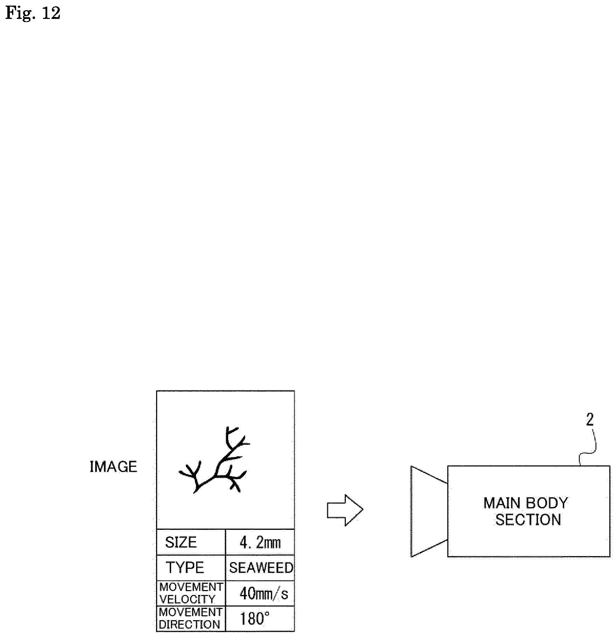

Fig. 12 is a diagram illustrating accumulated carbon amount estimation processing.
[Fig. 13]

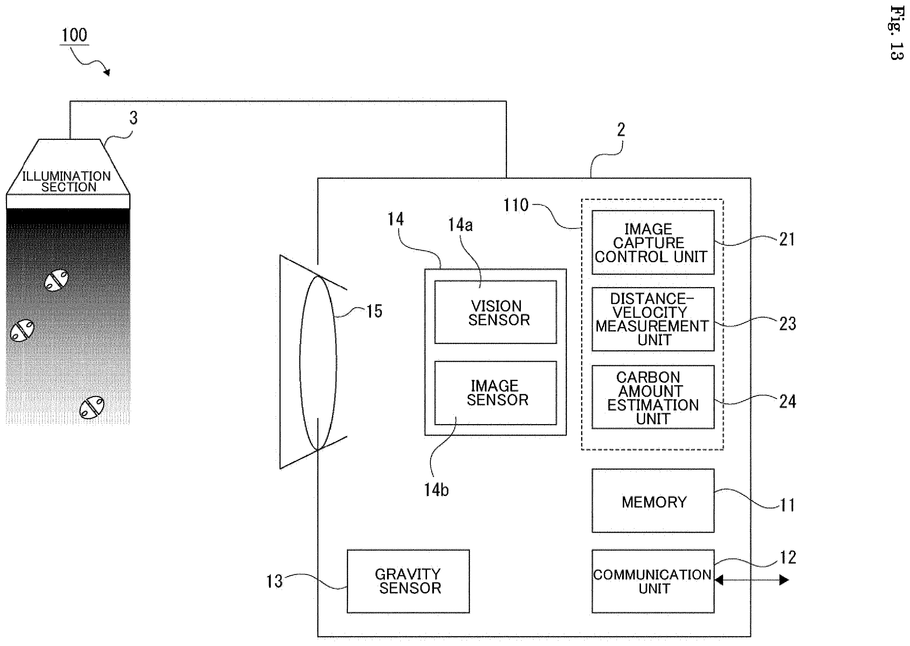

Fig. 13 is a diagram illustrating the configuration of a measurement device serving as a second embodiment of the present technique.
[Fig. 14]

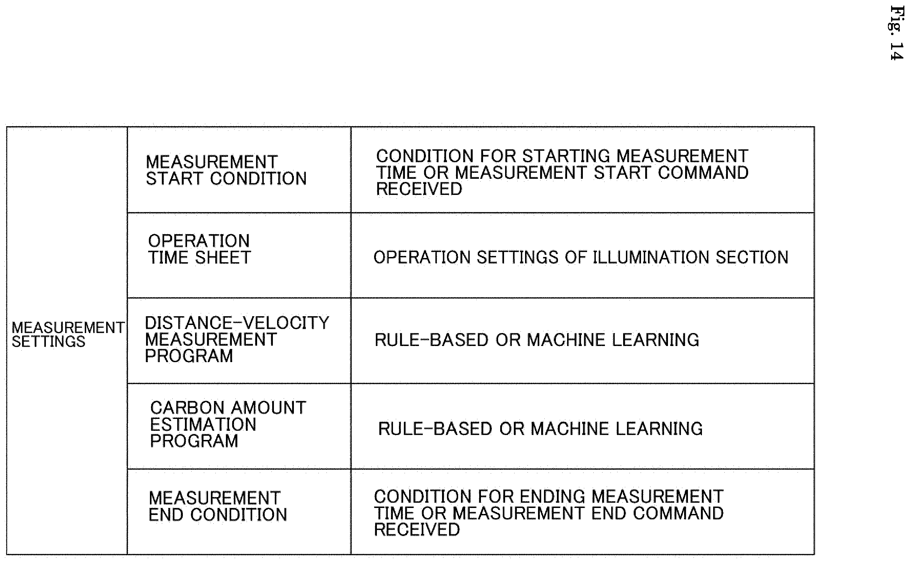

Fig. 14 is a diagram illustrating an example of measurement settings.
[Fig. 15]

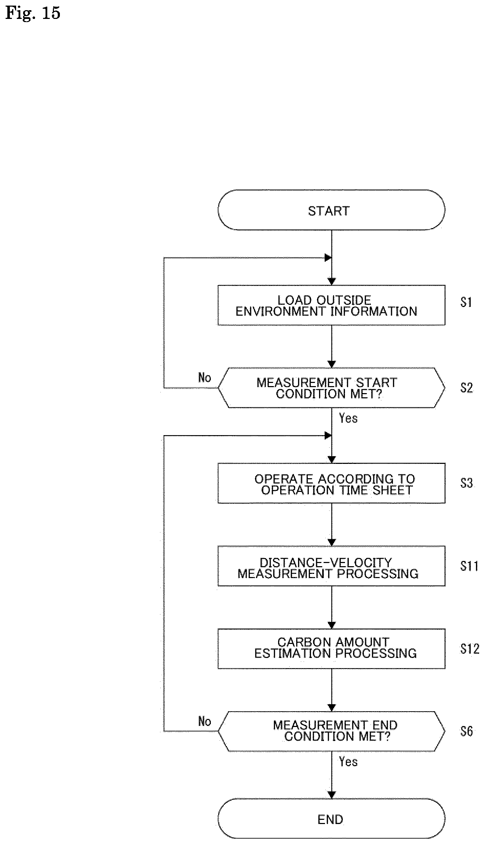

Fig. 15 is a flowchart illustrating a sequence of measurement processing.
[Fig. 16]

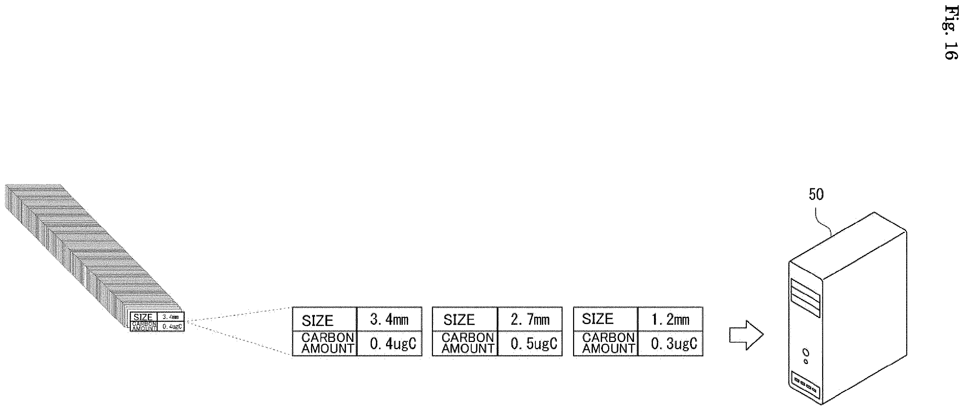

Fig. 16 is a diagram illustrating machine learning for carbon amount estimation processing.
[Fig. 17]

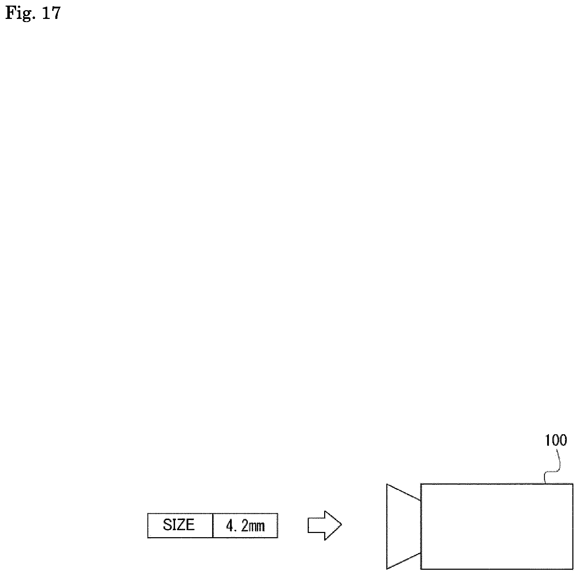

Fig. 17 is a diagram illustrating spatial carbon amount estimation processing.

[Fig. 18]

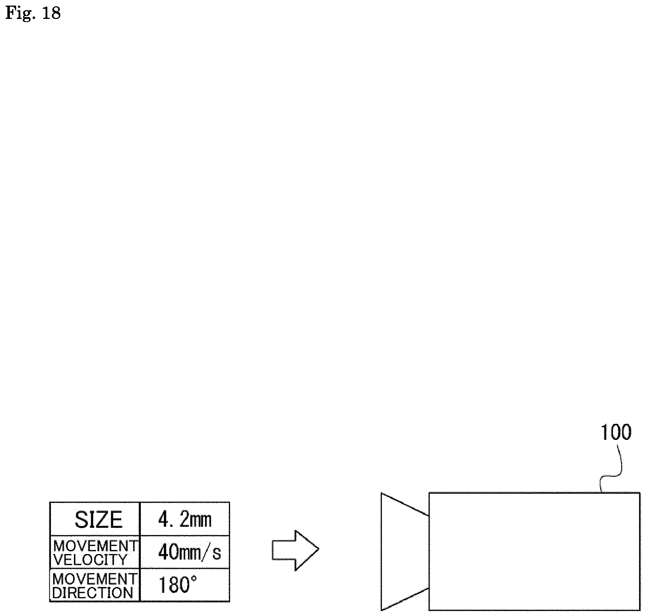

Fig. 18 is a diagram illustrating accumulated carbon amount estimation processing.

[Fig. 19]

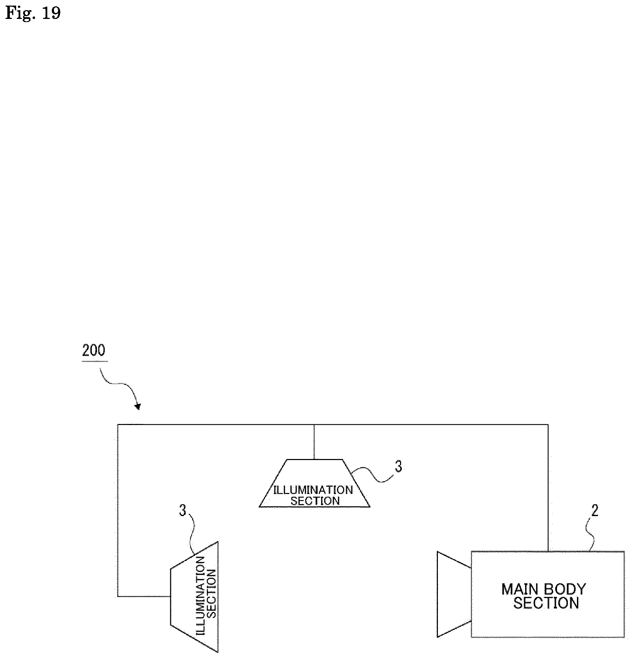

Fig. 19 is a diagram illustrating the configuration of a measurement device according to a variation.

[Fig. 20]

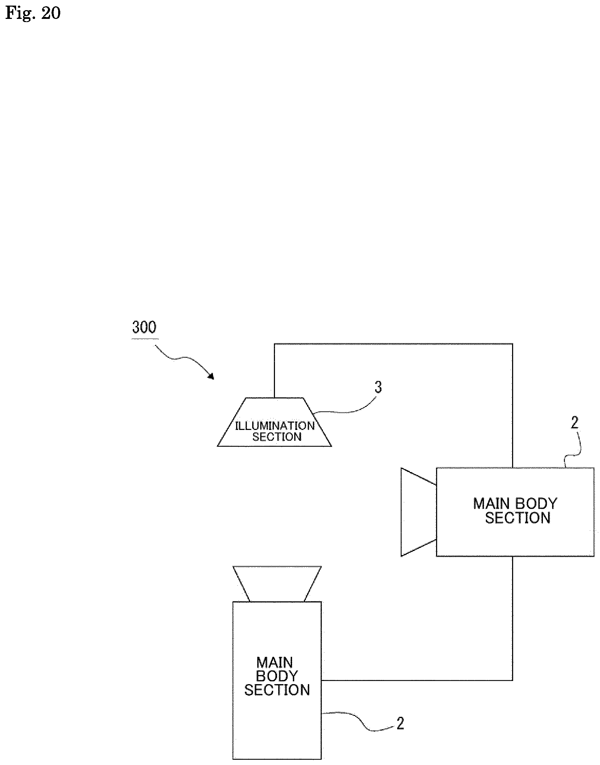

Fig. 20 is a diagram illustrating the configuration of a measurement device according to a variation.

[Description of Embodiments]

Hereinafter, embodiments will be described in the following order.

<1. First Embodiment>

[1.1 Configuration of Measurement Device]
[1.2 Target Object]
[1.3 Measurement Method of First Embodiment]
[1.4 Measurement Processing]
[1.5 Distance-Velocity Measurement Processing]
[1.6 Carbon Amount Estimation Processing]

<2. Second Embodiment>

[2.1 Configuration of Measurement Device]
[2.2 Measurement Processing]
[2.3 Machine Learning-Based Distance-Velocity Measurement Processing]
[2.4 Carbon Amount Estimation Processing]

<3. Example of Other Configuration of Measurement Device>
<4. Summary of Embodiments>
<5. Present Technique>

<1. First Embodiment>

[1.1 Configuration of Measurement Device]

The configuration of a measurement device 1, serving as a first embodiment of the present technique, will be described first.

The measurement device 1 is a device that takes microorganisms or microparticles present in water, such as ocean water, for example, as a target object, and estimates (measures) the amount of carbon in the water by estimating the amount of carbon in the target object.

Here, the microorganisms that are the target object are aquatic microorganisms such as phytoplankton, zooplankton, juvenile aquatic organisms present in the water, or the like. The microparticles that are the target object are microplastics, dirt, sand, marine snow, air bubbles, and the like. These are merely examples, however, and the target object may be another object.

Fig. 1 is a diagram illustrating the configuration of the measurement device 1 serving as the first embodiment. Fig. 2 is a diagram illustrating an image capture range 30 and a measurement direction.

As illustrated in Fig. 1, the measurement device 1 includes a main body section 2 and an illumination section 3. Note that the illumination section 3 may be provided in the main body section 2.

The main body section 2 includes a control unit 10, a memory 11, a communication unit 12, a gravity sensor 13, an image capture unit 14, and a lens 15.

The control unit 10 is configured including a microcomputer having, for example, a Central Processing Unit (CPU), Read Only Memory (ROM), and Random Access Memory (RAM), and controls the measurement device 1 as a whole. In the first embodiment, the control unit 10 functions as an image capture control unit 21, a class identification unit 22, a distance-velocity measurement unit 23, and a carbon amount estimation unit 24. Note that the image capture control unit 21, the class identification unit 22, the distance-velocity measurement unit 23, and the carbon amount estimation unit 24 will be described in detail later.

The control unit 10 also performs processing for reading out data stored in the memory 11, processing for storing data in the memory 11, and the transmission and reception of various types of data to and from external devices through the communication unit 12.

The memory 11 is constituted by a non-volatile memory. The communication unit 12 communicates data with external devices over wires or wirelessly. The gravity sensor 13 detects gravitational acceleration (a gravity direction) and outputs a detection result to the control unit 10. Note that the measurement device 1 need not include the gravity sensor 13.

The image capture unit 14 includes a vision sensor 14a, an image sensor 14b, or both. The vision sensor 14a is a sensor called a Dynamic Vision Sensor (DVS) or an Event-Based Vision Sensor (EVS). The vision sensor 14a captures a predetermined image capture range 30 in water through the lens 15. As illustrated in Fig. 2, in the following, the horizontal direction of the image capture range 30 may be referred to as an X-axis direction; the vertical direction of the image capture range 30, as a Y-axis direction; and an image capture direction of the image capture unit 14 (an optical axis direction), as a Z-axis direction. The Y-axis direction is assumed to substantially coincide with the gravity direction.

The vision sensor 14a is an asynchronous image sensor in which a plurality of pixels each having a photoelectric conversion element are arranged two-dimensionally, and a detection circuit that detects an address event in real time is provided for each pixel. Note that an "address event" is an event that occurs in accordance with an amount of light incident for each of addresses assigned to corresponding ones of the plurality of pixels arranged two-dimensionally, and is, for example, a current value of current based on a charge generated by the photoelectric conversion element, an amount of change in the current value exceeding a given threshold, or the like.

The vision sensor 14a detects whether or not an address event has occurred for each pixel, and if the occurrence of an address event is detected, reads out a pixel signal as pixel data from the pixel in which the address event occurred. In other words, the vision sensor 14a obtains the pixel data asynchronously, according to the amount of light incident on each of the pixels arranged two-dimensionally.

In the vision sensor 14a, pixel signal readout operations are executed for pixels in which the occurrence of an address event is detected. As such, the readout operations can be performed at much higher speed than in a synchronous image sensor, in which readout operations are executed for all pixels at a predetermined frame rate, and the amount of data to be read out as one frame is also smaller.

This makes it possible for the measurement device 1 to detect the movement of the target object more quickly by using the vision sensor 14a. The vision sensor 14a also makes it possible to reduce the amount of data and reduce power consumption.

The image sensor 14b is, for example, a CCD (Charge Coupled Device) or a CMOS (Complementary Metal-Oxide-Semiconductor) image sensor, and a plurality of pixels each having a photoelectric conversion element are arranged two-dimensionally. The image sensor 14b generates image data by capturing a predetermined image capture range 30 through the lens 15 at a constant interval according to a framerate. Note that in the measurement device 1, a zone plate, a pinhole plate, or a transparent plate can be used instead of the lens 15.

The vision sensor 14a and the image sensor 14b are disposed so as to capture substantially the same image capture range 30 through the lens 15. For example, a half mirror (not shown) may be disposed between the vision sensor 14a and the image sensor 14b and the lens 15, such that one part of the light divided by the half mirror is incident on the vision sensor 14a, and the other part is incident on the image sensor 14b.

The illumination section 3 is driven under the control of the control unit 10, and illuminates the image capture range 30 of the image capture unit 14. The illumination section 3 is capable of switching among different wavelengths of light to be emitted, e.g., emitting light at wavelengths of 10-nm intervals.

[1.2 Target Object]

Fig. 3 is a diagram illustrating a target object and movement of the target object. Note that in Fig. 3, an image of the target object is illustrated in the upper part, and the movement direction of the target object is indicated in the lower part by an arrow.

As illustrated in Fig. 3, the target object includes microorganisms, marine snow, seafloor sand, smoke, and bubbles.

It is known that some microorganisms exhibit motility when irradiated with light at a specific wavelength. Here, "motility" is inherent behavior of an organism in response to light (an external stimulus). Therefore, when a microorganism having motility is irradiated with light at a specific wavelength, the microorganism moves according to the motility.

Marine snow is particles, such as, for example, plankton waste, carcasses, decompositions thereof, and the like that are present in the ocean and which move so as to sink (in the direction of gravity) into the ocean.

Seafloor sand is, for example, particles such as sand deposited on the seafloor, and is moved in a swirling manner by seafloor current.

Smoke is, for example, a phenomenon in which hot water heated geothermally is ejected from hydrothermal vents on the seafloor. Hot water ejected from hydrothermal vents can reach hundreds of degrees, and because such water is rich in heavy metals and hydrogen sulfide as dissolved components, it reacts with the seawater and swirls upward as black or white smoke.

Air bubbles are, for example, natural gases such as methane or carbon dioxide that leak (are ejected) from the seafloor, carbon dioxide that leaks from artificially-injected reservoirs in carbon dioxide storage (CCS), and the like, which rise from the seafloor.

In this manner, even if the target object is not limited to microorganisms but also includes microparticles, some microparticles move in specific movement directions, and the measurement device 1 specifies microorganisms and microparticles for which the movement directions are known as the target object.

[1.3 Measurement Method of First Embodiment]

A measurement method (measurement processing) for a target object, serving as a first embodiment, will be described next.

The ocean is a lightless layer in which no sunlight can reach at a depth of about 150 m. The lightless layer occupies a major part of the open ocean, and many of the target objects described above are present therein. On the other hand, target objects are known to reflect or emit light at different wavelengths or intensities for each wavelength of light at which the target objects are irradiated.

Accordingly, the measurement device 1 specifies a type of the target object by irradiating the target object with light at different wavelengths and capturing images produced by the reflected light (or excitation light) under the assumption that the measurement is taken in the lightless layer, where sunlight does not reach. The measurement device 1 then estimates the amount of carbon for the target object for which the type has been specified.

Fig. 4 is a diagram illustrating an example of measurement settings. Fig. 5 is a diagram illustrating an example of an operation time sheet.

The control unit 10 takes the measurement according to predetermined measurement settings such as those illustrated in Fig. 4. The measurement settings specify a measurement start condition, an operation time sheet of the illumination section 3, an identification program, a distance-velocity measurement program, a carbon amount estimation program, and a measurement end condition.

The measurement start condition specifies a condition for starting the measurement, e.g., a time at which to start the measurement, the reception of a measurement start command input through the communication unit 12, or the like.

The operation time sheet specifies a time sheet for operating the illumination section 3. For example, according to the operation time sheet illustrated in Fig. 5, wavelengths are varied by 10 nm in a range of 400 nm to 700 nm, i.e., 400 nm, 410 nm, ..., 690 nm, and 700 nm, and the light is emitted so as to be off on either side of each of the wavelengths.

In this manner, the operation time sheet specifies the wavelength of the light and the timing at which the image capture range 30 is to be irradiated with light from the illumination section 3. Note that the illumination section 3 is provided with a period of being off, that is, not emitting light, in order to capture light when the target object is emitting light (is excited). This also has an effect in that the asynchronous vision sensor 14a can more easily detect wavelength-specific events by providing off ranges between the respective wavelengths.

The identification program specifies a program for identifying the type of the target object, e.g., a machine learning-based identification program, a rule-based identification program, or the like.

The distance-velocity measurement program specifies a program for measuring the distance, velocity, or the like of the target object, e.g., a machine learning-based distance-velocity measurement program, a rule-based distance-velocity measurement program, or the like.

The carbon amount estimation program specifies a program for estimating the amount of carbon, e.g., a machine learning-based carbon amount estimation program, a rule-based carbon amount estimation program, or the like.

The measurement end condition specifies a condition for ending the measurement, e.g., a time at which to end the measurement, the reception of a measurement end command input through the communication unit 12, or the like.

[1.4 Measurement Processing]

Fig. 6 is a flowchart illustrating a sequence of measurement processing. The control unit 10 executes the measurement processing illustrated in Fig. 6 by executing software (including the identification program, the distance-velocity measurement program, and the carbon amount estimation program) stored in the memory 11.

In step S1, the control unit 10 loads outside environment information, which will be described later. Then, in step S2, the control unit 10 determines whether the measurement start condition specified in the measurement settings is met. The control unit 10 then repeats step S1 and step S2 until the measurement start condition is met.

On the other hand, if the measurement start condition is met (Yes in step S2), in step S3, the image capture control unit 21 causes the illumination section 3 to emit light while switching among different wavelengths according to the operation time sheet specified in the measurement settings. In addition, each time the wavelength of the light emitted from the illumination section 3, and whether the light is on or off, are switched, the image capture control unit 21 causes the image capture unit 14 to capture the image capture range 30, and pixel data and image data are obtained as a result. Then, in step S4, the class identification unit 22 executes class identification processing.

In the class identification processing, the class identification unit 22 identifies (specifies) the type of the target object based on the image (pixel data and image data) captured by the image capture unit 14. For example, the class identification unit 22 identifies the type of the target object by deriving identification information from the image captured by the image capture unit 14 and comparing that identification information with definition information stored in the memory 11.

The definition information is provided for each target object, and is stored in the memory 11. The definition information includes the type of the target object, movement information, and image information.

The movement information is mainly information detected on the basis of the image captured by the vision sensor 14a, and is information based on the movement of the target object, as indicated in the lower part of Fig. 3. When the target object is a microorganism, the movement information is information such as a movement direction (positive or negative) relative to the light source, the trajectory, and the like. When the target object is a microparticle, the movement information is information such as a movement direction, a trajectory, and the like.

The image information is mainly information detected on the basis of the image captured by the image sensor 14b, and is information indicating the external form of the target object. Note that the image information may be information detected on the basis of the image captured by the vision sensor 14a.

The definition information may also include the gravity direction detected by the gravity sensor 13, the outside environment information obtained through the communication unit 12, and the like. Depth, position coordinates (latitude and longitude of the measurement point, plane rectangular coordinates), electrical conductivity, temperature, ph, the concentration of gas (e.g., methane, hydrogen, helium), the concentration of metal (e.g., manganese, iron), and the like are conceivable as the outside environment information.

The class identification unit 22 detects an object present in the image capture range 30 based on the image (pixel data) captured by the vision sensor 14a. For example, the class identification unit 22 creates an image (frame data) based on the pixel data input within a predetermined period, and detects a pixel group within a predetermined range in which motion is detected in that image as a single object.

In addition, the class identification unit 22 tracks objects among a plurality of frames through pattern matching or the like. Then, the class identification unit 22 derives the movement direction and trajectory of the object as identification information based on a result of tracking the object.

Note that the period during which the class identification unit 22 generates the image from the pixel data may be the same as or shorter than the period during which the image sensor 14b obtains the image data (the framerate).

In addition, the class identification unit 22 extracts, for the object for which the identification information has been derived, an image part corresponding to the object, in the image data input from the image sensor 14b. The class identification unit 22 then derives external features as identification information through image analysis based on the extracted image part. Note that a publicly-known method can be used for the image analysis, and will therefore not be described here.

The class identification unit 22 identifies whether an object is a target object by comparing the wavelength of the light emitted from the illumination section 3, the identification information (movement direction, trajectory, and external features) derived for the detected object, and the definition information according to the specified identification program. Here, if, for example, the derived identification information of the object is within a range indicated in the definition information of the target object, the class identification unit 22 identifies that object as being of the type indicated in the definition information.

The definition information is stored in the memory 11 using different methods depending on the identification program. For example, in a rule-based identification program, the definition information is set in advance by the user and stored in the memory 11. In a machine learning-based identification program, the definition information is generated and updated through the machine learning in a learning mode, and is stored in the memory 11.

After this, the class identification unit 22 stores the result of identifying the detected target object, the image part of the target object captured by the image sensor 14b, and the like in the memory 11, transmits these items to an external device through the communication unit 12, and the like.

In step S5, the distance-velocity measurement unit 23 executes distance-velocity measurement processing that measures a distance to and a velocity of the target object based on the type of the target object identified by the class identification unit 22. The distance-velocity measurement processing of step S5 will be described in detail later.

Then, in step S6, the carbon amount estimation unit 24 executes carbon amount estimation processing that estimates the amount of carbon in the water. The carbon amount estimation processing of step S6 will be described in detail later.

Then, in step S7, the control unit 10 determines whether the measurement end condition is met. The control unit 10 repeats step S3 to step S6 until the measurement end condition is met, and once the end condition is met (Yes in step S6), the determination processing ends.

[1.5 Distance-Velocity Measurement Processing]

The distance-velocity measurement processing will be described next. As described above, the distance-velocity measurement unit 23 performs the distance-velocity measurement processing based on a rule-based or a machine learning-based distance-velocity measurement program in step S5.

The rule-based distance-velocity measurement processing and the machine learning-based distance-velocity measurement processing will be described here with specific examples.

[1.5.1 Rule-Based Distance-Velocity Measurement Processing]

Fig. 7 is a diagram illustrating the rule-based distance-velocity measurement processing. In the rule-based distance-velocity measurement processing, a focal length f of the vision sensor 14a is stored in the memory 11 as known information.

Statistical information (an average size H) for each target object is stored in the memory 11. This is registered by the user as a database in advance.

Then, when the type of the target object is identified from the image based on the pixel data, the distance-velocity measurement unit 23 reads out the average size H for that type and the focal length f of the vision sensor 14a from the memory 11. The distance-velocity measurement unit 23 then calculates a length s, in a lengthwise direction, of an image 42 of the target object captured on an image capture plane 40 of the vision sensor 14a by, for example, multiplying the actual length of each pixel by the number of pixels in which the image 42 appears.

The distance-velocity measurement unit 23 then calculates a distance D in the image capture direction (in the Z direction) from the measurement device 1 to a target object 41 using Formula (1).

$$D = fH/s \ ... \ (1)$$

In this manner, the distance-velocity measurement unit 23 calculates (measures) the actual distance D from the measurement device 1 to the target object 41 every time an image based on the pixel data is obtained (every time a target object is detected from an image). The distance-velocity measurement unit 23 also calculates a velocity in the image capture direction (the Z-axis direction) for the target object 41 that is being tracked between successive images based on an interval at which the images are obtained and the distance D in each image.

The distance-velocity measurement unit 23 further calculates the velocity of the target object in the X-axis direction and the Y-axis direction based on the interval at which images are obtained, the number of pixels by which the target object has moved between the images (i.e., the distance moved on the image capture plane 40), and the distance D in the image capture direction in each image.

By doing so, the distance-velocity measurement unit 23 calculates the velocity of the target object in each of the three axial directions.

As described above, in the rule-based distance-velocity measurement processing, the distance-velocity measurement unit 23 measures a distance to and a velocity of a target object based on statistical information (an average size) for each target object.

[1.5.2 Machine Learning-Based Distance-Velocity Measurement Processing]

Fig. 8 is a diagram illustrating images serving as supervisory data. Fig. 9 is a diagram illustrating a deep learning model.

In the machine learning-based distance-velocity measurement processing, machine learning is performed using images serving as supervisory data, as illustrated in Fig. 8, to generate a model (architecture) for distance-velocity measurement processing.

Specifically, images of known target objects captured by the vision sensor 14a are prepared in advance for five patterns of distances from the measurement device 1 to the target object in the image capture direction, namely 1 mm, 5 mm, 10 mm, 100 mm, and 200 mm, and 31 patterns of wavelengths of emitted light, namely every 10 nm from 400 nm to 700 nm, for a total of 153 patterns.

Then, for each prepared image, the distance-velocity measurement unit 23 detects a pixel group within a predetermined range in which motion is detected as a target object and resizes that pixel group to 32 pixels × 32 pixels to generate an image serving as supervisory data, as illustrated in Fig. 8.

Note that Fig. 8 illustrates only some of the images serving as supervisory data. Here, in water, the attenuation rate of light at about 500 nm is low, whereas the attenuation rate of light at wavelengths lower than about 500 nm and wavelengths higher than about 500 nm increases with the further away from about 500 nm the wavelength is.

In addition, the arrival rate of light decreases as the distance from the measurement device 1 to the target object increases.

Accordingly, as illustrated in Fig. 8, in an image in which the target object is captured, the target object becomes clearer the closer the target object is to the measurement device 1, and the closer the wavelength of the emitted light is to 500 nm. The target object loses clarity or disappears completely the further the target object is from the measurement device 1, and the farther the wavelength of the emitted light is from 500 nm.

When the images serving as the supervisory data are resized, the distance-velocity measurement unit 23 causes the supervisory data constituted by these images to undergo machine learning using a deep neural network, as illustrated in Fig. 9. The model is constituted by, for example, five convolutional layers (Conv1 to Conv5), three pooling layers (Max Pooling), and two fully connected layers (FC). As a result of this machine learning, a model in which a one-dimensional classification vector having five elements, from Distance 1 mm to Distance 200 mm, is ultimately output, is generated and stored in the memory 11.

Machine learning in such a deep neural network is performed for each target object, and a model for each target object is generated and stored in the memory 11.

Then, when the type of the target object is identified by the class identification unit 22 (step S4), the distance-velocity measurement unit 23 reads out the model for the identified type from the memory 11. The distance-velocity measurement unit 23 also resizes the target object part of the image captured by the vision sensor 14a to 32 pixels × 32 pixels, and inputs the resized image into the read-out model. As a result, a value of the one-dimensional classification vector having five elements, from Distance 1 mm to Distance 200 mm, is output. The distance-velocity measurement unit 23 then outputs (measures) the element having the highest value among the five elements (one of Distance 1 mm to Distance 200 mm) as the distance of the target object in the image capture direction.

The distance-velocity measurement unit 23 also calculates a velocity in the image capture direction (the Z-axis direction) for the target object that is being tracked between successive images based on an interval at which the images are obtained and the distance in the image capture direction in each image.

The distance-velocity measurement unit 23 further geometrically calculates the velocity of the target object in the X-axis direction and the Y-axis direction based on the interval at which images are obtained, the number of pixels by which the target object has moved between the images (i.e., the distance moved on the image capture plane 40), and the distance D in the image capture direction in each image.

By doing so, the distance-velocity measurement unit 23 calculates the velocity of the target object in each of the three axial directions.

The distance-velocity measurement unit 23 also calculates the size (length: H) of the target object in the lengthwise direction by substituting the focal length f of the vision sensor 14a, the distance (D) of the target object in the image capture direction, and the length s, in the lengthwise direction, of the image 42 of the target object captured on the image capture plane 40, into Formula (1).

As described above, in the machine learning-based distance-velocity measurement processing, the distance-velocity measurement unit 23 measures a size, a distance, and a velocity of a target object based on a learning result learned in advance for each type of target object.

[1.6 Carbon Amount Estimation Processing]

Incidentally, to reduce greenhouse gases that contribute to global warming, it is necessary to observe changes in carbon, which is the source of the greenhouse gas carbon dioxide, over the long term. Some carbon dioxide is absorbed by oceans (into water). Some of the carbon dioxide absorbed by oceans is also captured as carbon by phytoplankton through photosynthesis. Furthermore, the carcasses and feces of phytoplankton, or planktonic organisms that preyed on phytoplankton (zooplankton, juvenile organisms, and the like), accumulate in deep waters as marine snow. The marine snow that accumulates at the bottom of the water becomes a huge carbon sink effective for thousands of years.

As such, estimating the amount of carbon present in the water, or the amount of carbon that has accumulated at the bottom of the water, can be expected to help reduce greenhouse gases.

Accordingly, the carbon amount estimation unit 24 estimates the amount of carbon in the water by performing the carbon amount estimation processing in step S6. The carbon amount estimation processing includes spatial carbon amount estimation processing that estimates the amount of carbon present in a predetermined volume at a specific timing, and accumulated carbon amount estimation processing that estimates the amount of carbon that has accumulated at the bottom of the water in a predetermined period.

In the carbon amount estimation processing, the amount of carbon is estimated by a rule-based or machine learning-based carbon amount estimation program, as described above. The machine learning-based carbon amount estimation processing will be described in detail here.

Fig. 10 is a diagram illustrating machine learning for the carbon amount estimation processing. As illustrated in Fig. 10, in the learning mode of the carbon amount estimation processing, a large amount of supervisory data in which target object input information is labeled with amounts of carbon is prepared, and a computer 50 generates a carbon amount estimation model. Images, sizes, and types of target objects are set as the target object input information, for example.

The computer 50, which includes a CPU, then generates the carbon amount estimation model by performing machine learning using the supervisory data through a known algorithm. The carbon amount estimation model generated here takes the target object input information as an input, and outputs an amount of carbon estimated from that input information. The generated carbon amount estimation model is then stored in the memory 11 of the measurement device 1 in advance. Note that the computer 50 may be the measurement device 1.

Fig. 11 is a diagram illustrating the spatial carbon amount estimation processing. In the spatial carbon amount estimation processing, the carbon amount estimation unit 24 estimates a total amount of carbon for all the target objects identified in a single instance of image capturing.

Specifically, as illustrated in Fig. 11, the carbon amount estimation unit 24 derives an amount of carbon for each target object by obtaining an image, a size, and a type of the target object identified or measured by the class identification unit 22 and the distance-velocity measurement unit 23 as input information, and inputting the obtained input information into the carbon amount estimation model.

Then, by adding the estimated amounts of carbon of the target objects, the carbon amount estimation unit 24 calculates a total amount of carbon for all the target objects identified in a single instance of image capturing. Here, the image capture conditions such as the image capture range and the focal length of the image capture unit 14 are known in advance, and thus the volume of the image capture range that can be captured by the image capture unit 14 is also known.

Accordingly, the carbon amount estimation unit 24 calculates an instantaneous amount of carbon (ugC/L) per unit of volume by dividing the total amount of carbon of all the target objects identified in a single instance of image capturing by the known volume of the image capture range.

The amount of carbon calculated here is the amount of carbon held by all the target objects identified in a single instance of image capturing, and therefore takes on a value indicating the amount of carbon held in the water.

Fig. 12 is a diagram illustrating the accumulated carbon amount estimation processing.

In the accumulated carbon amount estimation processing, the carbon amount estimation unit 24 extracts, from among target objects captured in a predetermined period of time such as one minute, a target object that has moved downward based on the movement direction of the target object. Note that in Fig. 12, the movement direction is indicated as an angle on a predetermined vertical plane that takes 0° as vertically upward, and here, a target object having a movement direction greater than 90° and less than 270° is extracted. In other words, only target objects that will accumulate at the bottom of the water are extracted here.

Then, the carbon amount estimation unit 24 obtains an image, a size, and a type of the extracted target object as the input information, and uses the carbon amount estimation model to estimate the amount of carbon in each target object moving downward. In addition, the carbon amount estimation unit 24 calculates a time required for each target object to accumulate at the bottom of the water based on the movement velocity of the corresponding target object. Note that the distance from the position where the measurement device 1 is provided to the bottom of the water is known.

Then, on the basis of the amount of carbon for each extracted target object and the time required for the target object to accumulate at the bottom of the water, the carbon amount estimation unit 24 calculates the total amount of carbon in the target objects that reach the bottom of the water in one day, for example, as the amount of carbon that accumulates each day (ugC/day).

Note that in the rule-based carbon amount estimation processing, a table in which amounts of carbon are defined for the input information is stored in advance in the memory 11, and when the input information is input, the carbon amount estimation unit 24 estimates the amount of carbon through verification with the table stored in the memory 11.

<2. Second Embodiment>

[2.1 Configuration of Measurement Device]

Fig. 13 is a diagram illustrating the configuration of a measurement device 100 serving as a second embodiment of the present technique. As illustrated in Fig. 13, the measurement device 100 differs from the measurement device 1 serving as the first embodiment in that a control unit 110 does not function as the class identification unit 22, but the rest of the configuration is the same as the measurement device 1.

On the basis of the image captured by the vision sensor 14a, the measurement device 100 measures a distance to and a velocity of a target object in an image capture direction without specifying a type of the target object.

Fig. 14 is a diagram illustrating an example of measurement settings. The control unit 110 takes the measurement according to predetermined measurement settings such as those illustrated in Fig. 14. The measurement settings specify a measurement start condition, an operation time sheet of the illumination section 3, a distance-velocity measurement program, a carbon amount estimation program, and a measurement end condition.

The measurement start condition specifies a condition for starting the measurement, e.g., a time at which to start the measurement, the reception of a measurement start command input through the communication unit 12, or the like.

The operation time sheet specifies a time sheet for operating the illumination section 3. For example, according to the operation time sheet illustrated in Fig. 5, the wavelengths are varied by 10 nm in a range of 400 nm to 700 nm, i.e., 400 nm, 410 nm, ..., 690 nm, and 700 nm, and the light is emitted while being turned on and off repeatedly.

The distance-velocity measurement program specifies a program for measuring the distance, velocity, or the like of the target object, e.g., a machine learning-based distance-velocity measurement program, a rule-based distance-velocity measurement program, or the like.

The carbon amount estimation program specifies a program for estimating the amount of carbon, e.g., a machine learning-based carbon amount estimation program, a rule-based carbon amount estimation program, or the like.

The measurement end condition specifies a condition for ending the measurement, e.g., a time at which to end the measurement, the reception of a measurement end command input through the communication unit 12, or the like.

In this manner, the measurement settings in the second embodiment differ from the measurement settings in the first embodiment in that no identification program is provided.

[2.2 Measurement Processing]

Fig. 15 is a flowchart illustrating a sequence of measurement processing. The control unit 110 executes the measurement processing illustrated in Fig. 15 by executing software (including the distance-velocity measurement program and the carbon amount estimation program) stored in the memory 11.

In step S1, the control unit 110 loads outside environment information. Then, in step S2, the control unit 10 determines whether the measurement start condition specified in the measurement settings is met. The control unit 110 then repeats step S1 and step S2 until the measurement start condition is met.

On the other hand, if the measurement start condition is met (Yes in step S2), in step S3, the image capture control unit 21 causes the illumination section 3 to emit light while switching among different wavelengths according to the operation time sheet specified in the measurement settings. In addition, each time the wavelength of the light emitted from the illumination section 3, and whether the light is on or off, are switched, the image capture control unit 21 causes the image capture unit 14 to capture the image capture range 30, and pixel data and image data are obtained as a result.

Then, in step S11, the distance-velocity measurement unit 23 detects an object present in the image capture range as a target object based on an image based on pixel data, and executes distance-velocity measurement processing that measures a size, a distance, and a velocity of that target object. The distance-velocity measurement processing of step S11 will be described in detail later.

Then, in step S12, the carbon amount estimation unit 24 executes carbon amount estimation processing that estimates the amount of carbon in the water. The carbon amount estimation processing of step S12 will be described in detail later.

Then, in step S6, the control unit 10 determines whether an end condition for ending the determination processing is met. The control unit 10 repeats step S3 to step S6 until the end condition for ending the determination processing is met, and once the end condition for ending the purpose-based measurement operation processing is met (Yes in step S6), the determination processing ends.

[2.3 Machine Learning-Based Distance-Velocity Measurement Processing]

As described above, the distance-velocity measurement unit 23 performs the distance-velocity measurement processing based on a rule-based or a machine learning-based distance-velocity measurement program in step 511.

The machine learning-based distance-velocity measurement processing will be described here with specific examples.

Like the measurement device 1, the measurement device 100 creates a deep learning model, as illustrated in Fig. 9.

In the first embodiment, a model was generated for each target object, but in the second embodiment, rather than generating a model for each target object, only one model trained in advance, independent of the type of the target object, is generated.

Specifically, five patterns of distances from the measurement device 1 to the target object in the image capture direction, namely 1 mm, 5 mm, 10 mm, 100 mm, and 200 mm, and 31 patterns of wavelengths of emitted light, namely every 10 nm from 400 nm to 700 nm, for a total of 153 patterns, are taken, and a number of images equivalent to the number of types of target object captured by the vision sensor 14a while varying the target object, multiplied by the total of 153 patterns, are prepared.

Then, for each prepared image, the distance-velocity measurement unit 23 detects a pixel group within a predetermined range in which motion is detected as a target object and resizes that pixel group to 32 pixels × 32 pixels to generate an image serving as supervisory data, as illustrated in Fig. 8.

When the images serving as the supervisory data are resized, the distance-velocity measurement unit 23 causes the supervisory data constituted by these images to undergo machine learning using a deep neural network, and stores the generated model in the memory 11, as illustrated in Fig. 9.

The distance-velocity measurement unit 23 then resizes the target object part of the image captured by the vision sensor 14a to 32 pixels × 32 pixels, and inputs the resized image into the model read out from the memory 11. As a result, a value of the one-dimensional classification vector having five elements, from Distance 1 mm to Distance 200 mm, is output. The distance-velocity measurement unit 23 then outputs (measures) the element having the highest value among the five elements (Distance 1 mm to Distance 200 mm) as the distance of the target object in the image capture direction.

The distance-velocity measurement unit 23 also calculates (measures) a velocity in the image capture direction (the Z-axis direction) for the target object that is being tracked between successive images based on an interval at which the images are obtained and the distance in the image capture direction in each image.

The distance-velocity measurement unit 23 further calculates a velocity in the image capture direction (the Z-axis direction) for the target object that is being tracked between successive images based on an interval at which the images are obtained and the distance in the image capture direction in each image.

The distance-velocity measurement unit 23 further calculates the velocity of the target object in the X-axis direction and the Y-axis direction based on the interval at which images are obtained, the number of pixels by which the target object has moved between the images (i.e., the distance moved on the image capture plane 40), and the distance D in the image capture direction in each image.

By doing so, the distance-velocity measurement unit 23 calculates the velocity of the target object in each of the three axial directions.

The distance-velocity measurement unit 23 also calculates the size (length: H) of the target object in the lengthwise direction by substituting the focal length f of the vision sensor 14a, the distance (D) of the target object in the image capture direction, and the length s, in the lengthwise direction, of the image 42 of the target object captured on the image capture plane 40, into Formula (1).

As described above, in the machine learning-based distance-velocity measurement processing, the distance-velocity measurement unit 23 measures a size, a distance, and a velocity of a target object based on a learning result learned in advance for each type of target object.

Accordingly, in the second embodiment, there are fewer models than in the first embodiment, and thus the amount of data can be reduced. In the second embodiment, the measurement accuracy for the distance is lower, but the calculation time is also shorter.

[2.4 Carbon Amount Estimation Processing]

Fig. 16 is a diagram illustrating machine learning for the carbon amount estimation processing. As described above, in step S12, the carbon amount estimation unit 24 executes the carbon amount estimation processing based on a rule-based or machine learning-based carbon amount estimation program.

The machine learning-based carbon amount estimation processing will be described here with specific examples.

Like the measurement device 1, the measurement device 100 creates a carbon amount estimation model.

In the first embodiment, the carbon amount estimation model was generated using the type, size, and image information of the target object as input information, but in the second embodiment, the carbon amount estimation model is generated without using the type and image information of the target object as input information.

In other words, as illustrated in Fig. 16, the target object size is set as the target object input information, for example. The computer 50, which includes a CPU, then generates the carbon amount estimation model by performing machine learning through a known algorithm, using a large amount of supervisory data including sizes and amounts of carbon of target objects. The generated carbon amount estimation model is then stored in the memory 11 of the measurement device 1 in advance.

Fig. 17 is a diagram illustrating the spatial carbon amount estimation processing. As illustrated in Fig. 17, in the spatial carbon amount estimation processing, the carbon amount estimation unit 24 estimates an amount of carbon for all the target objects identified in a single instance of image capturing.

Specifically, the carbon amount estimation unit 24 estimates the amount of carbon in each target object using the carbon amount estimation model, taking the size of the target object as input information. Then, by adding the estimated amounts of carbon of the target objects, the carbon amount estimation unit 24 calculates a total amount of carbon for all the target objects identified in a single instance of image capturing.

In addition, the carbon amount estimation unit 24 calculates an instantaneous amount of carbon per unit of volume (ugC/L) by dividing the total amount of carbon of all the target objects identified in a single instance of image capturing by the known volume of the image capture range.

Fig. 18 is a diagram illustrating the accumulated carbon amount estimation processing.

In the accumulated carbon amount estimation processing, the carbon amount estimation unit 24 extracts, from among target objects captured in, for example, one minute, a target object that moves downward based on the movement direction of the target object.

Then, the carbon amount estimation unit 24 estimates the amount of carbon in each target object using the carbon amount estimation model, taking the size of the extracted target object as input information. In addition, the carbon amount estimation unit 24 calculates a time required for each target object to accumulate at the bottom of the water based on the movement velocity of the corresponding target object.

Then, on the basis of the amount of carbon for each extracted target object and the time required for the target object to accumulate at the bottom of the water, the carbon amount estimation unit 24 calculates the total amount of carbon in the target objects that reach the bottom of the water in one day, for example, as the amount of carbon that accumulates each day (ugC/day).

<3. Example of Other Configuration of Measurement Device>

The embodiment is not limited to the specific examples described above, and configurations as various variations can be adopted.

In the foregoing embodiments, the measurement device 1 is provided with a single illumination section 3. However, the number of illumination sections 3 is not limited to one, and a plurality may be provided.

Fig. 19 is a diagram illustrating the configuration of a measurement device 200 according to a variation. As illustrated in Fig. 19, the measurement device 200 according to the variation includes one main body section 2 and two illumination sections 3. The two illumination sections 3 are arranged so as to be capable of emitting light in directions orthogonal to each other, and are capable of irradiating the image capture range with light at mutually different wavelengths. Note that in addition to emitting the light in directions orthogonal to each other, the illumination sections 3 may emit the light at angles or parallel, in accordance with the measurement to be taken.

In such a measurement device 200, light at different wavelengths can be emitted from the two illumination sections 3, and thus identification information of the target object (microorganism) indicating motilities for light at different wavelengths can be derived from a single measurement, which enables efficient measurement.

Fig. 20 is a diagram illustrating the configuration of a measurement device 300 according to a variation. As illustrated in Fig. 20, the measurement device 300 according to the variation includes two main body section 2 and one illumination section 3. The two main body sections 2 are disposed such that images can be captured from directions orthogonal to each other. Note that in addition to being disposed so as to capture images from directions orthogonal to each other, the main body sections 2 may be disposed such that images can be captured at angles or parallel, in accordance with the measurement to be taken.

In this measurement device 300, images can be captured by the two main body sections 2 (the image capture units 14), which makes it possible to detect the movement of the target object in three dimensions, and take measurements more efficiently.

Note that when two main body sections 2 are provided, one of the main body sections 2 may include only the image capture unit 14.

In addition, in the foregoing embodiments, the image capture unit 14 includes the vision sensor 14a and the image sensor 14b. However, the image capture unit 14 may include only one of the vision sensor 14a or the image sensor 14b. In addition, the image capture unit 14 may include a Single Photon Avalanche Diode (SPAD) sensor instead of the vision sensor 14a and the image sensor 14b.

In addition, the methods for identifying or measuring the type, size, movement direction, distance, velocity, and movement velocity of the target object described in the foregoing embodiments are only examples, and the type, size, movement direction, distance, velocity, and movement velocity of the target object may be identified or measured by various known methods.

In the foregoing embodiments, one or more of the type, size, and image information of the target object was taken as information pertaining to the target object, and the amount of carbon was estimated using information pertaining to the target object as input information. However, the information pertaining to the target object may include not only the type, size, and image information of the target object, but also other information such as the movement velocity, the movement direction, and the like.

<4. Summary of Embodiments>

As described above, the measurement device 1 according to an embodiment includes the image capture control unit 21 that causes the image capture unit 14 to capture an image of a predetermined image capture range in water, and the carbon amount estimation unit 24 that estimates an amount of carbon in the water based on the image captured by the image capture unit 14.

This makes it possible for the measurement device 1 to estimate an amount of carbon based on an image captured by the image capture unit 14.

This in turn makes it possible for the measurement device 1 to observe changes in carbon over the long term.

In the measurement device 1 according to the present technique described above, the image capture unit 14 includes the vision sensor 14a that obtains pixel data asynchronously according to an amount of light incident on each of a plurality of pixels arranged two-dimensionally.

This makes it possible to read out only the pixel data of pixels where an event has occurred, and estimate the amount of carbon based on that pixel data.

Accordingly, the measurement device 1 can reduce the computational cost of image processing by capturing images at high speed, reducing power consumption, and automatically separating the background.

In the measurement device 1 according to the present technique described above, the carbon amount estimation unit 24 may estimate an amount of carbon present in the image capture range.

This makes it possible to estimate the amount of carbon contained (dissolved) in the water.

In the measurement device 1 according to the present technique described above, the carbon amount estimation unit 24 may estimate the amount of carbon that has accumulated at the bottom of the water.

This makes it possible to ascertain the amount of carbon that has been captured at the bottom of the water.

In the measurement device 1 according to the present technique described above, the carbon amount estimation unit 24 may estimate the amount of carbon that has accumulated at the bottom of the water per predetermined length of time.

This makes it possible to ascertain transitions in the amount of carbon that has been captured at the bottom of the water.

The measurement device 1 according to the present technique described above may include a measurement unit that measures a size of a target object captured by the image capture unit 14 (the distance-velocity measurement unit 23), and the carbon amount estimation unit 24 may estimate the amount of carbon based on the size of the target object.

The proportion of carbon contained in the target object present in the water is known to some extent, and thus by estimating the amount of carbon based on the size of the target object, the amount of carbon can be estimated through simple processing.

The measurement device 1 according to the present technique described above may include the class identification unit 22 that identifies a type of the target object captured by the image capture unit 14, and the carbon amount estimation unit 24 may estimate the amount of carbon based on the type and size of the target object.

The proportion of carbon for each type of target object is known, and thus estimating the amount of carbon based on the type and size of the target object makes it possible to accurately estimate the amount of carbon.

In the measurement device 1 according to the present technique described above, the class identification unit 22 may extract an image part of the target object captured by the image capture unit 14, and the carbon amount estimation unit may estimate the amount of carbon based on the image part, the type, and the size of the target object.

Further using the image part of the target object makes it possible to more accurately estimate the amount of carbon.

The measurement device 1 according to the present technique described above may include a direction measurement unit (the class identification unit 22) that measures a movement direction of the target object captured by the image capture unit 14, and the carbon amount estimation unit 24 may specify a target object that has accumulated at the bottom of the water based on the movement direction of the target object.

This makes it possible to accurately estimate the amount of carbon that has accumulated at the bottom of the water based on the target object that has accumulated at the bottom of the water.

The measurement device 1 according to the present technique described above may include a velocity measurement unit (the distance-velocity measurement unit 23) that measures a movement velocity of the target object captured by the image capture unit 14, and the carbon amount estimation unit 24 may specify a target object that has accumulated at the bottom of the water per predetermined length of time based on the movement direction and the movement velocity of the target object.

This makes it possible to accurately estimate the amount of carbon that has accumulated at the bottom of the water per predetermined length of time based on the target object that has accumulated at the bottom of the water per predetermined length of time.

In a measurement method according to the present technique described above, an image capture unit is caused to capture an image of a predetermined image capture range in water, and an amount of carbon is estimated on the basis of the image captured by the image capture unit.

A program according to the present technique described above causes an information processing device to perform processing of causing an image capture unit to capture an image of a predetermined image capture range in water, and estimating an amount of carbon based on the image captured by the image capture unit.

The program can be recorded in advance in an HDD serving as a recording medium embedded in a device such as a computer device or a ROM or the like in a microcomputer that includes a CPU.

Alternatively, the program can be stored (recorded) temporarily or perpetually on a removable recording medium such as a flexible disc, a compact disc read-only memory (CD-ROM), a magneto optical (MO) disc, a digital versatile disc (DVD), a Blu-ray Disc (registered trademark), a magnetic disk, a semiconductor memory, or a memory card. The removable recording medium can be provided as so-called package software.

The program can be installed from the removable recording medium on a personal computer or the like and can also be downloaded from a download site via a network such as a local area network (LAN) or the Internet.

Note that the effects described in the present specification are merely exemplary and not intended to be limiting, and other effects may be provided as well.

<5. Present Technique>

The present technique can also be configured as follows.

(1) A measurement device including:

an image capture control unit that causes an image capture unit to capture an image of a predetermined image capture range in water; and
a carbon amount estimation unit that estimates an amount of carbon based on the image captured by the image capture unit.

(2) The measurement device according to (1), wherein the image capture unit includes a vision sensor that obtains pixel data asynchronously according to an amount of light incident on each of a plurality of pixels arranged two-dimensionally.

(3) The measurement device according to (1) or (2), wherein the carbon amount estimation unit estimates an amount of carbon present in the image capture range.

(4) The measurement device according to any one of (1) to (3),
wherein the carbon amount estimation unit estimates the amount of carbon that has accumulated at a bottom of the water.

(5) The measurement device according to (4),
wherein the carbon amount estimation unit estimates the amount of carbon that has accumulated at the bottom of the water per predetermined length of time.

(6) The measurement device according to any one of (1) to (5), further including:

a measurement unit that measures a size of a target object captured by the image capture unit,
wherein the carbon amount estimation unit estimates the amount of carbon based on the size of the target object.

(7) The measurement device according to (6), further including:

a class identification unit that identifies a type of the target object captured by the image capture unit,
wherein the carbon amount estimation unit estimates the amount of carbon based on the type and the size of the target object.

(8) The measurement device according to (7),

wherein the class identification unit extracts an image part of the target object captured by the image capture unit, and the carbon amount estimation unit estimates the amount of carbon based on the image part, the type, and the size of the target object.

(9) The measurement device according to (5), further including:

a direction measurement unit that measures a movement direction of a target object captured by the image capture unit, wherein the carbon amount estimation unit specifies a target object that accumulates at the bottom of the water based on the movement direction of the target object.

(10) The measurement device according to (9), further including:

a velocity measurement unit that measures a movement velocity of the target object captured by the image capture unit, wherein the carbon amount estimation unit specifies a target object that accumulates at the bottom of the water per predetermined length of time based on the movement direction and the movement velocity of the target object.

(11) A measurement method including:

causing an image capture unit to capture an image of a predetermined image capture range in water; and estimating an amount of carbon based on the image captured by the image capture unit.

(12) A program that causes a measurement device to execute processing of:

causing an image capture unit to capture an image of a predetermined image capture range in water; and estimating an amount of carbon based on the image captured by the image capture unit.

[Reference Signs List]

| | |
|---|---|
| 1 | Measurement device |
| 3 | Illumination section |
| 10 | Control unit |
| 14 | Image capture unit |
| 14a | Vision sensor |
| 14b | Image sensor |
| 21 | Image capture control unit |

| | |
|---|---|
| 22 | Class identification unit |
| 23 | Distance-velocity measurement unit |
| 24 | Carbon amount estimation unit |

**Claims**

1. A measurement device comprising:

an image capture control unit that causes an image capture unit to capture an image of a predetermined image capture range in water; and a carbon amount estimation unit that estimates an amount of carbon based on the image captured by the image capture unit.

2. The measurement device according to claim 1, wherein the image capture unit includes a vision sensor that obtains pixel data asynchronously according to an amount of light incident on each of a plurality of pixels arranged two-dimensionally.

3. The measurement device according to claim 1, wherein the carbon amount estimation unit estimates an amount of carbon present in the image capture range.

4. The measurement device according to claim 1, wherein the carbon amount estimation unit estimates the amount of carbon that has accumulated at a bottom of the water.

5. The measurement device according to claim 4, wherein the carbon amount estimation unit estimates the amount of carbon that has accumulated at the bottom of the water per predetermined length of time.

6. The measurement device according to claim 1, further comprising:

a measurement unit that measures a size of a target object captured by the image capture unit, wherein the carbon amount estimation unit estimates the amount of carbon based on the size of the target object.

7. The measurement device according to claim 6, further comprising:

a class identification unit that identifies a type of the target object captured by the image capture unit, wherein the carbon amount estimation unit estimates the amount of carbon based on the type and the size of the target object.

8. The measurement device according to claim 7,

wherein the class identification unit extracts an image part of the target object captured by the image capture unit, and
the carbon amount estimation unit estimates the amount of carbon based on the image part, the type, and the size of the target object.

9. The measurement device according to claim 5, further comprising:

a direction measurement unit that measures a movement direction of a target object captured by the image capture unit,
wherein the carbon amount estimation unit specifies a target object that accumulates at the bottom of the water based on the movement direction of the target object.

10. The measurement device according to claim 9, further comprising:

a velocity measurement unit that measures a movement velocity of the target object captured by the image capture unit,
wherein the carbon amount estimation unit specifies a target object that accumulates at the bottom of the water per predetermined length of time based on the movement direction and the movement velocity of the target object.

11. A measurement method comprising:

causing an image capture unit to capture an image of a predetermined image capture range in water; and
estimating an amount of carbon based on the image captured by the image capture unit.

12. A program that causes a measurement device to execute processing of:

causing an image capture unit to capture an image of a predetermined image capture range in water; and
estimating an amount of carbon based on the image captured by the image capture unit.

Fig. 1

Fig. 2

Fig. 3

Fig. 4

| MEASUREMENT SETTINGS | MEASUREMENT START CONDITION | CONDITION FOR STARTING MEASUREMENT TIME OR MEASUREMENT START COMMAND RECEIVED |
|---|---|---|
| | OPERATION TIME SHEET | OPERATION SETTINGS OF ILLUMINATION SECTION |
| | IDENTIFICATION PROGRAM | RULE-BASED OR MACHINE LEARNING |
| | DISTANCE-VELOCITY MEASUREMENT PROGRAM | RULE-BASED OR MACHINE LEARNING |
| | CARBON AMOUNT ESTIMATION PROGRAM | RULE-BASED OR MACHINE LEARNING |
| | MEASUREMENT END CONDITION | CONDITION FOR ENDING MEASUREMENT TIME OR MEASUREMENT END COMMAND RECEIVED |

Fig. 5

| TIME | 0 | 1 | 2 | 3 | 4 | 5 | · · · · · | | 690 nm | OFF | 700 nm | · · · · · |
|------|-----|--------|-----|--------|-----|--------|-----------|---|--------|-----|--------|---|
| WAVELENGTH | OFF | 400 nm | OFF | 410 nm | OFF | 420 nm | · · · | | | | | |

Fig. 6

```
                    ╭─────────────────────╮
                    │       START         │
                    ╰─────────────────────╯
                              │
    ┌─────────────────────────┼
    │                         ▼
    │          ┌─────────────────────────────┐
    │          │      LOAD OUTSIDE           │  S1
    │          │  ENVIRONMENT INFORMATION    │
    │          └─────────────────────────────┘
    │                         │
    │  No      ╱─────────────────────────────╲
    └─────────   MEASUREMENT START           │  S2
               ╲  CONDITION MET?             ╱
                └─────────────────────────────┘
                          │ Yes
    ┌─────────────────────┼
    │                     ▼
    │          ┌─────────────────────────────┐
    │          │   OPERATE ACCORDING TO      │  S3
    │          │    OPERATION TIME SHEET     │
    │          └─────────────────────────────┘
    │                     │
    │                     ▼
    │          ┌─────────────────────────────┐
    │          │   CLASS IDENTIFICATION      │  S4
    │          │      PROCESSING             │
    │          └─────────────────────────────┘
    │                     │
    │                     ▼
    │          ┌─────────────────────────────┐
    │          │   DISTANCE-VELOCITY         │  S5
    │          │ MEASUREMENT PROCESSING      │
    │          └─────────────────────────────┘
    │                     │
    │                     ▼
    │          ┌─────────────────────────────┐
    │          │    CARBON AMOUNT            │  S6
    │          │ ESTIMATION PROCESSING       │
    │          └─────────────────────────────┘
    │                     │
    │  No      ╱─────────────────────────────╲
    └─────────   MEASUREMENT END             │  S7
               ╲  CONDITION MET?             ╱
                └─────────────────────────────┘
                          │ Yes
                          ▼
                    ╭─────────────────────╮
                    │        END          │
                    ╰─────────────────────╯
```

Fig. 7

Fig. 8

Fig. 9

Fig. 10

| | |
|---|---|
| SIZE | 3.4mm |
| TYPE | SEAWEED |
| CARBON AMOUNT | 0.4ugC |

| | |
|---|---|
| SIZE | 2.7mm |
| TYPE | PELLET |
| CARBON AMOUNT | 0.5ugC |

| | |
|---|---|
| SIZE | 1.2mm |
| TYPE | AMORPHOUS |
| CARBON AMOUNT | 0.3ugC |

IMAGE

| | |
|---|---|
| SIZE | 3.4mm |
| TYPE | SEAWEED |
| CARBON AMOUNT | 0.4ugC |

50

Fig. 11

IMAGE

| | |
|---|---|
| SIZE | 4. 2mm |
| TYPE | SEAWEED |

2

MAIN BODY
SECTION

Fig. 12

IMAGE

| SIZE | 4. 2mm |
|------|--------|
| TYPE | SEAWEED |
| MOVEMENT VELOCITY | 40mm/s |
| MOVEMENT DIRECTION | 180° |

2

MAIN BODY
SECTION

Fig. 13

EP 4 394 354 A1

Fig. 14

| MEASUREMENT SETTINGS | MEASUREMENT START CONDITION | CONDITION FOR STARTING MEASUREMENT TIME OR MEASUREMENT START COMMAND RECEIVED |
|---|---|---|
| | OPERATION TIME SHEET | OPERATION SETTINGS OF ILLUMINATION SECTION |
| | DISTANCE-VELOCITY MEASUREMENT PROGRAM | RULE-BASED OR MACHINE LEARNING |
| | CARBON AMOUNT ESTIMATION PROGRAM | RULE-BASED OR MACHINE LEARNING |
| | MEASUREMENT END CONDITION | CONDITION FOR ENDING MEASUREMENT TIME OR MEASUREMENT END COMMAND RECEIVED |

Fig. 15

```
              ┌─────────────────────┐
              │        START        │
              └─────────────────────┘
                         │
                         ▼
              ┌─────────────────────┐
              │    LOAD OUTSIDE      │  S1
              │ ENVIRONMENT INFORMATION │
              └─────────────────────┘
                         │
                         ▼
      No     ╱─────────────────────╲
    ◄────────   MEASUREMENT START      S2
              ╲    CONDITION MET?    ╱
                         │
                        Yes
                         │
                         ▼
              ┌─────────────────────┐
              │  OPERATE ACCORDING TO │  S3
              │ OPERATION TIME SHEET │
              └─────────────────────┘
                         │
                         ▼
              ┌─────────────────────┐
              │   DISTANCE-VELOCITY  │  S11
              │ MEASUREMENT PROCESSING │
              └─────────────────────┘
                         │
                         ▼
              ┌─────────────────────┐
              │   CARBON AMOUNT      │  S12
              │ ESTIMATION PROCESSING │
              └─────────────────────┘
                         │
                         ▼
      No     ╱─────────────────────╲
    ◄────────   MEASUREMENT END        S6
              ╲    CONDITION MET?    ╱
                         │
                        Yes
                         │
                         ▼
              ┌─────────────────────┐
              │         END         │
              └─────────────────────┘
```

Fig. 16

| SIZE | 3.4mm |
|------|-------|
| CARBON AMOUNT | 0.4ugC |

| SIZE | 2.7mm |
|------|-------|
| CARBON AMOUNT | 0.5ugC |

| SIZE | 1.2mm |
|------|-------|
| CARBON AMOUNT | 0.3ugC |

50

| SIZE | 3.4mm |
|------|-------|
| CARBON AMOUNT | 0.4ugC |

Fig. 17

| SIZE | 4.2mm |

⟹

100

Fig. 18

| SIZE | 4. 2mm |
|---|---|
| MOVEMENT VELOCITY | 40mm/s |
| MOVEMENT DIRECTION | 180° |

100

Fig. 19

200

ILLUMINATION
SECTION

3

3

ILLUMINATION
SECTION

MAIN BODY
SECTION

2

Fig. 20

300

ILLUMINATION
SECTION

3

2

MAIN BODY
SECTION

MAIN
BODY
SECTION

2

## INTERNATIONAL SEARCH REPORT

| International application No. |
|---|
| **PCT/JP2022/012747** |

**A. CLASSIFICATION OF SUBJECT MATTER**

***G01N 21/27*** (2006.01)i
FI: G01N21/27 A

According to International Patent Classification (IPC) or to both national classification and IPC

**B. FIELDS SEARCHED**

Minimum documentation searched (classification system followed by classification symbols)

G01N21/00-21/01; G01N21/17-21/61; G06N3/00-3/12; G06N7/08-99/00; G01W1/00-1/18

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus (JDreamIII), JST7580 (JDreamIII), JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 6-304546 A (HITACHI LTD) 01 November 1994 (1994-11-01) paragraphs [0037]-[0041], fig. 2-3 | 1, 3, 6-8, 11-12 |
| A | paragraphs [0037]-[0041], fig. 2-3 | 2, 4-5, 9-10 |
| Y | 宮井 博, 外3名, 植物プランクトンの形態に基づく種類別生物量の簡易推定法, 日本プランクトン学会売, 1988, vol. 35, no. 2, pp. 121-126, entire text, all drawings in particular, "Method 1. A simple measuring method for the cell volume of phytoplankton", "Result 2. Phytoplankton biomass ", table 2, (MIYAI, Hiroshi et al. A Simple Method for the Estimation of Phytoplankton Biomass Based on Cell Morphology. Bulletin of Plankton Society of Japan.) | 1, 3, 6-8, 11-12 |
| A | CN 112362544 A (NANJING JIZE INFORMATION TECHNOLOGY CO., LTD.) 12 February 2021 (2021-02-12) | 1-12 |

☐ Further documents are listed in the continuation of Box C.  ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **18 May 2022** | **31 May 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

| | International application No. |
|---|---|
| | **PCT/JP2022/012747** |

| Patent document cited in search report | Publication date (day/month/year) | Patent family member(s) | Publication date (day/month/year) |
|---|---|---|---|
| JP 6-304546 A | 01 November 1994 | (Family: none) | |
| CN 112362544 A | 12 February 2021 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2019165687 A